Europäisches Patentamt

European Patent Office

Office européen des brevets

(11) Veröffentlichungsnummer: **0 128 404 B1**

(12) **EUROPÄISCHE PATENTSCHRIFT**

(45) Veröffentlichungstag der Patentschrift:
09.08.89

(51) Int. Cl.⁴: **F 01 K 23/06**, F 02 C 3/28, C 07 C 53/08, C 07 C 69/15

(21) Anmeldenummer: 84105781.3

(22) Anmeldetag: 21.05.84

(54) **Kraftwerk mit einer integrierten Kohlevergasungsanlage.**

(30) Priorität: 03.06.83 DE 3320227

(43) Veröffentlichungstag der Anmeldung:
19.12.84 Patentblatt 84/51

(45) Bekanntmachung des Hinweises auf die Patenterteilung: 09.08.89 Patentblatt 89/32

(84) Benannte Vertragsstaaten:
AT BE CH DE FR GB IT LI LU NL SE

(56) Entgegenhaltungen:
EP--A-- 0 028 474
DE--A-- 2 024 301
DE--A-- 2 425 939
DE--A-- 2 827 498
FR--A-- 877 792
GB--A-- 2 075 124
GB--A-- 2 084 973
US--A-- 4 277 416

(73) Patentinhaber: Siemens Aktiengesellschaft Berlin und München
Wittelsbacherplatz 2
D-8000 München 2 (DE)

(72) Erfinder: Schiffers, Ulrich, Dr. Dipl.-Ing.
Moritzbergstrasse 1
D-8501 Eckental (DE)
Erfinder: Müller, Rainer, Dr. Dipl.-Ing.
Herdegenplatz 1
D-8520 Erlangen (DE)

## Beschreibung

Die Erfindung bezieht sich auf ein Kraftwerk mit einer integrierten Kohlevergasungsanlage, mit einer am Kohlevergaser angeschlossenen Wärmetauscher- und Gasreinigungsanlage, mit einem an die Wärmetauscher- und Gasreinigungsanlage angeschlossenen Gasturbinen und Dampfkraftwerksteil und mit einer Methanolsyntheseanlage, wobei das überschüssige Syntheseabgas aus der Methanolsynthese der Brennkammer des Gasturbinenkraftwerksteils zuleitbar ist.

Die DE-OS 31 14 984 offenbart eine Kraftwerksanlage, bei der eine Gasturbine von einer Kohlevergasungsanlage mit Synthesegas versorgt wird. Die Gasturbine treibt einen elektrischen Generator an. Die Abhitze der Gasturbine wird bei dieser Kraftwerksanlage zur Dampferzeugung herangezogen. Mit dem Dampf wird eine Dampfturbine und ein weiterer elektrischer Generator angetrieben. Zusätzlich ist bei dieser Kraftwerksanlage vorgesehen, daß ein Teil des erzeugten Synthesegases einer Methanolsyntheseanlage zugeführt wird. Das erzeugte Methanol wird bei dieser Kraftwerksanlage gespeichert und zum Ausgleich von Lastspitzen zusätzlich zum Synthesegas in der Gasturbine mit verbrannt. Diese Kraftwerksanlage erlaubt es in Zeiten von Schwachlast verstärkt Methanol zu erzeugen und das so erzeugte Methanol, sofern es nicht zum Ausgleich von Spitzenlasten benötigt wird, als Rohstoff zu verkaufen. Abgesehen davon, daß bei häufigem Ausfahren von Lastspitzen nur ein geringer Teil des erzeugten Methanols frei verfügbar ist, liegen die Gestehungskosten für das Methanol nicht wesentlich unter denen entsprechender kraftwerksunabhängiger Herstellverfahren.

Der vorliegenden Erfindung liegt die Aufgabe zugrunde, die Wirtschaftlichkeit eines solchen Kraftwerks zu verbessern und dabei Chemierohstoffe möglichst preiswert herzustellen.

Bei einem Kraftwerk der eingangs genannten Art ist daher erfindungsgemäß das in der Methanolsyntheseanlage erzeugte Methanol wie auch das Syntheseabgas der Methanolsynthese zumindest teilweise einer Essigsäuresyntheseanlage, das Abgas aus dieser Essigsäuresyntheseanlage der Brennkammer des Gasturbinenkraftwerkteils zuleitbar und ist eine Gaszerlegungsanlage an der Gaszuleitung zum Methanolsynthesereaktor angeschlossen, deren wasserstoffreiche Fraktion dem in den Methanolsynthesereaktor strömenden Gasstrom beimischbar und deren kohlenmonoxydreiche Fraktion sowohl dem Essigsäurereaktor als auch der Brennkammer des Gasturbinenkraftwerkteils zuleitbar ist. Infolge der Integration dieser beiden aneinander gekoppelten Teilanlagen für eine Methanolsynthese und eine Essigsäuresynthese in das Kraftwerk wird durch die Verwendung des dort erzeugten Synthesegases zur Methanolherstellung und durch die Einspeisung sowohl eines Teiles des Methanols und des Abgases aus der Methanolsynthese in die Essigsäuresynthese erreicht, daß diese Chemierohstoffe kostengünstiger herstellbar sind. Infolge der Verbrennung der überschüssigen Syntheseabgase in der Brennkammer der Gasturbine kann der Aufwand für die Herstellung stöchiometrischer Verhältnisse der Ausgangsgase vermindert werden, ohne daß dadurch der Energieinhalt der nicht vollständig umgesetzten Syntheseabgase verloren geht. Dabei verhilft die Gaszerlegungsanlage dazu, das Verhältnis von Wasserstoff zu Kohlenmonoxyd in dem dem Methanolsynthesereaktor angebotenen Synthesegas näher an das angestrebte stöchiometrische Verhältnis heranführen. Hierdurch erhöht sich die Ausbeute an Methanol. Zugleich kann so auch der Kohlenmonoxydanteil des dem Essigsäurereaktor zugeführten Gases erhöht werden, wodurch wiederum ein größerer Anteil des erzeugten Methanols in Essigsäure umsetzbar ist.

Bei einem Kraftwerk der eingangs genannten Art kann alternativ das in der methanolsyntheseanlage erzeugte Methanol wie auch das Synthesegas der Methanolsynthese zumindest teilweise erfindungsgemäß einer Anlage zur Erzeugung von Vinylacetat und das Abgas aus dieser Anlage zur Erzeugung von Vinylacetat der Brennkammer des Gasturbinenkraftwerkteils zuleitbar sein. Auch hierbei werden die nicht vollständig umgesetzten Syntheseabgase der vorgeschalteten, mit nicht stöchiometrisch zusammengesetztem Reingas betriebenen, Methanolsynthese ausnutzbar. Die Erstellung von Vinylacetat im Anschluß an die Methanoldurchlaufsynthese ist deshalb besonders zweckmäßig, weil diese Abgase der Methanoldurchlaufsynthese in etwa die Zusammensetzung besitzen, die für die Vinylacetatsynthese benötigt wird. Hierdurch könnte sogar die bei der Essigsäureherstellung zweckmäßigerweise vorzusehende Gaszerlegungsanlage eingespart werden. Zugleich kann auch hier wieder auf Bausteine verzichtet werden, die sonst bei fehlender Verbrennungsmöglichkeit der Syntheseabgase erforderlich würden. Somit läßt sich auf diese Weise ein weiterer gegebenenfalls auf dem Markt gewinnbringend veräußerbarer Chemierohstoff preisgünstig erstellen. Dabei tragen die so erstellten Chemierohstoffe auch noch dazu bei, den Kohlevergaser auch bei Schwachlastbetrieb mit Nennlast zu betreiben.

In besonders zweckmäßiger Weiterbildung der Erfindung kann die Methanolsyntheseanlage rezirkulationsfrei, das heißt ohne Rezirkulationsleitung und Kreislaufverdichter, ausgebildet sein. Hierdurch wird der Investitionsaufwand um diese, infolge der Integration in das Kraftwerk, nicht mehr benötigten Bausteine vermindert. Auch vermindert sich so der Energieaufwand für die Rezirkulation des nicht vollständig umgesetzten Syntheseabgases. Beides führt zu einer preisgünstigeren Herstellung der Chemierohstoffe.

Eine noch stärkere Annäherung der Zusammensetzung des dem Methanolsynthesereaktors angebotenen Synthesegases an das stöchiometri-

sche Verhältnis läßt sich erreichen, wenn in zweckmäßiger Ausgestaltung der Erfindung dem Methanolsynthesereaktor eine Wasserelektrolyseanlage zugeordnet ist, deren Sauerstoffleitung an den Kohlevergaser und deren Wasserstoffleitung an die zum Methanolsynthesereaktor führende Gaszuleitung angeschlossen ist. Hierdurch läßt sich sowohl Stoffausbeute bedeutend verbessern, als auch die dem Kohlevergaser vorgeschaltete Luftzerlegungsanlage entlasten.

Weitere Einzelheiten der Erfindung werden anhand von zwei in den Figuren dargestellten Ausführungsbeispielen erläutert. Es zeigen :

Fig. 1 ein erfindungsgemäßes Kraftwerk mit einer verbundenen Anlage zur Herstellung von Methanol und Essigsäure und

Fig. 2 ein anderes erfindungsgemäßes Kraftwerk mit einer verbundenen Anlage zur Herstellung von Methanol und Vinylacetat.

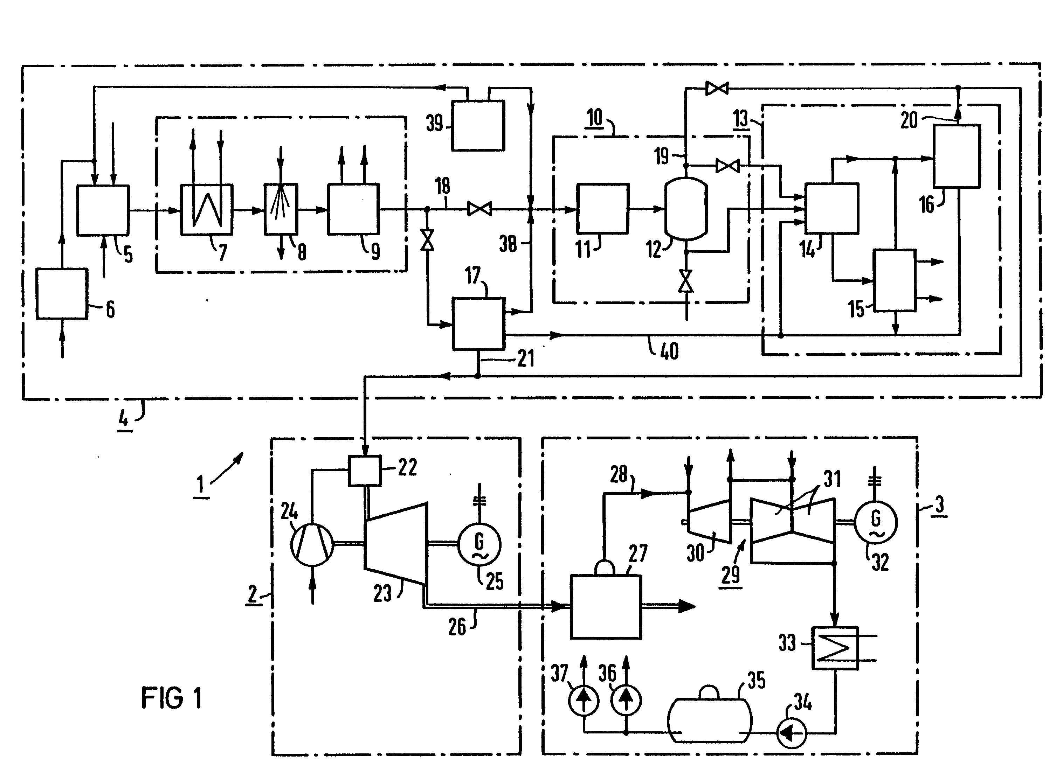

Die Fig. 1 zeigt in einer schematischen Darstellung ein Kraftwerk 1, welches sich aus einem Gasturbinenkraftwerksteil 2, einen Dampfkraftwerksteil 3 und einer Anlage 4 zur Herstellung von Chemierohstoffen zusammensetzt. Die Anlage 4 zur Herstellung von Chemierohstoffen beinhaltet einen Kohlevergaser 5 mit zugeordneter Luftzerleungsanlage 6, eine dem Kohlevergaser 5 nachgeschaltete Wärmetauscheranlage 7 für das Rohgas, eine Gasentstaubungsanlage 8, eine Gasreinigungsanlage 9, eine Methanolsyntheseanlage 10 mit einem Methanolsynthesereaktor 11 und einem Methanolabscheider 12 sowie eine Essigsäuresyntheseanlage 13 mit einem Essigsäurereaktor 14, einer Destillationskolonne 15 für die flüssigen Reaktionsprodukte und einem Waschsystem 16 für die gasförmigen Reaktionsprodukte des Essigsäurereaktors. Außerdem ist eine Gaszerlegungsanlage 17 an die die Gasreinigungsanlage 9 verlassende Reingasleitung 18 angeschlossen. Die Abgasleitung 19 des Methanolabscheiders 12, die Abgasleitung 20 des dem Essigsäurereaktor nachgeschalteten Waschsystems 16 für die gasförmigen Reaktionsprodukte und die Abgasleitung 21 für die Restgase der Gaszerlegungsanlage 17 sind zusammen an der Brennkammer 22 des Gasturbinenkraftwerksteils 2 angeschlossen.

Die Gasturbine 23 des Gasturbinenkraftwerkteiles 2 treibt sowohl einen Luftverdichter 24 für die Verbrennungsluft, als auch einen Generator 25 an. An die Abgasleitung 26 der Gasturbine ist ein Abhitzekessel 27 angeschlossen.

An der Dampfleitung 28 des Abhitzekessels ist die Dampfturbine 29 des Dampfkraftwerkteils 3 angeschlossen. Die Dampfturbine 29 besteht im Ausführungsbeispiel aus einem Hochdruckteil 30 und Niederdruckteil 31. Sie ist mit einem Generator 32 gekuppelt. An dem Niederdruckteil der Dampfturbine schließen sich ein Kondensator 33, eine Kondensatpumpe 34, ein Speisewasserbehälter 35 und verschiedene Speisewasserpumpen 36, 37 an.

Dem Kohlevergaser 5 wird gemahlene Kohle, Sauerstoff aus der vorgeschalteten Luftzerlegungsanlage 6 sowie Prozeßdampf zugeleitet.

Das im Kohlevergaser erzeugte heiße Rohgas gibt zunächst seine Wärme in der Wärmetauscheranlage 7 ab. Mit der Wärme wird Dampf erzeugt, der als Frischdampf dem Dampfkraftwerksteil 3 zugeleitet werden kann. In der an die Wärmetauscheranlage anschließenden Entstaubungsanlage 8 werden Staubpartikel aus dem Rohgas ausgeschieden, schließlich werden Kohlendioxyd und Schwefelwasserstoff in der Gasreinigungsanlage 9 vom Rohgas abgetrennt. Das die Gasreinigungsanlage verlassende Reingas wird über die Reingasleitung 18 sowohl der Methanolsyntheseanlage 10 als auch der Gaszerlegungsanlage 17 zugeleitet. Im Methanolsynthesereaktor 11 wird das Reingas teilweise zu Methanol umgesetzt. Die Umsetzung ist auch deshalb unvollständig, weil das Verhältnis von $H_2$ zu CO beim Reingas im Bereich von 0,5 bis 1 statt im stöchiometrischen Verhältnis von 2 liegt. Um das dem Methanolsynthesereaktor zugeleitete Reingas in seiner Zusammensetzung näher an das für die Methanolsynthesereaktion erforderliche stöchiometrische Verhältnis heranzubringen, ist an die die Gasreinigungsanlage 9 verlassende Reingasleitung 18 eine Gaszerlegungsanlage 17 angeschlossen. Der in der Gaszerlegungsanlage abgetrennte Wasserstoff wird über eine Rückführleitung 38 wieder dem Reingas beigemischt, das in den Methanolsynthesereaktor strömt. Dadurch erhöht sich dessen Wasserstoffanteil. Das hat wiederum zur Folge, daß ein höherer Anteil des angebotenen Reingas-Wasserstoffgemisches zu Methanol umgewandelt werden kann. Die genaue Zusammensetzung des dem Methanolsynthesereaktor zugeleiteten Gasgemisches läßt sich über die beiden in der Reingasleitung 18 eingebauten Regelventile steuern.

In Zeiten der Schwachlast, wenn dem Kraftwerk 1 wenig elektrische Energie abgenommen wird, kann durch Zuschaltung von Wasserelektrolysezellen 39 zusätzlich Wasserstoff und Sauerstoff hergestellt werden. Während der Sauerstoff dem Kohlevergaser 5 zugeleitet werden kann, kann dann der Wasserstoff dem Reingas zusätzlich beigemischt werden um damit eine weitere Annäherung an das für die Methanolsynthese angestrebte stöchiometrische Verhältnis zu erreichen.

Einem Teil des im Methanolabscheider 12 abgeschiedenen Methanols wird bei dieser Anlage 4 zur Herstellung von Chemierohstoffen zusammen mit einem Teil der Abgase der Methanolsynthese dem Essigsäurereaktor 14 zugeleitet. Um das zuvor erzeugte Methanol in Essigsäure umzuwandeln, braucht nur noch verhältnismäßig wenig Kohlenmonoxyd zusätzlich zu jedem Kohlenmonoxyd, das in dem Abgas des Methanolabscheiders enthalten ist, zugeführt zu werden. Soll möglichst viel Essigsäure hergestellt werden, so genügt es, wenn ein verhältnismäßig kleiner Anteil des Reingases über die Gaszerlegungsanlage 17 geleitet wird, um von der Gaszerlegungsanlage jene zusätzliche Kohlenmonoxydgasmenge zu bekommen, die bei Essigsäuresynthese noch benötigt wird. Sie wird über die Leitung 40 dem Essigsäurereaktor 14 zugeleitet. Wird dagegen ein größerer Anteil des Reingases aus der Reingasleitung 18 über die Gaszerlegungsanlage 17 geleitet, so kann mehr Wasserstoffgas der Methanolsyntheseanlage 10 zugeführt und auch mehr Methanol erzeugt werden. Die aus der Zerlegung zur Verfügung stehende Kohlenmonoxydgasmenge, die nicht zur Umsetzung von zumindest einem Teil des Methanols in Essigsäure verbraucht wird, kann daher über die Abgasleitung 19 der Brennkammer 22 der Gasturbine 23 zugeleitet werden. Auf diese Weise ist es möglich, den Anteil des Verhältnisses der Stromerzeugung zur Herstellung von Chemierohstoffen in gewissen Grenzen an die momentane Bedarfssituation anzupassen.

In der Destillationskolonne 15 wird die Essigsäure von der übrigen flüssigen Reaktionsprodukten getrennt. Im Waschsystem 16 werden die gasförmigen Reaktionsprodukte aufgearbeitet. Dabei wird nicht umgesetztes Methanol in den Essigsäurereaktor 14 zurückgeführt. Die Abgase des Waschsystems werden zusammen mit den Abgasen der Methanolsyntheseanlage 10 und dem Restgas aus der Gaszerlegungsanlage 17 dem Brenner 22 der Gasturbine 23 zugeleitet. Sie werden dort zusammen mit dem vom Verdichter 24 gelieferten Luft verbrannt. Diese Möglichkeit die Abgase in der Gasturbine zu verbrennen, führt dazu, daß man die sonst bei der Methanol- und Essigsäureherstellung verwendete Konvertierung einsparen kann. Auch fallen so keine schwer verwertbaren flüssigen Rückstände an.

Die heißen Abgase der Gasturbine 23 werden über die Abgasleitung 26 in den Abhitzekesse 27 geleitet. Ihre Abwärme wird zur Dampferzeugung verwendet. Der im Abhitzekesses 27 erzeugte Dampf, sowie zusätzlicher in der Wärmetauscheranlage 7 erzeugter Dampf werden der Dampfturbine 29 zugeleitet. Der Prozeßdampf, der als Vergasungsdampf und als Heizdampf für einzelne Stufen des Herstellverfahrens benötigt wird, wird der Dampfturbine an den entsprechenden Druckstufen entnommen.

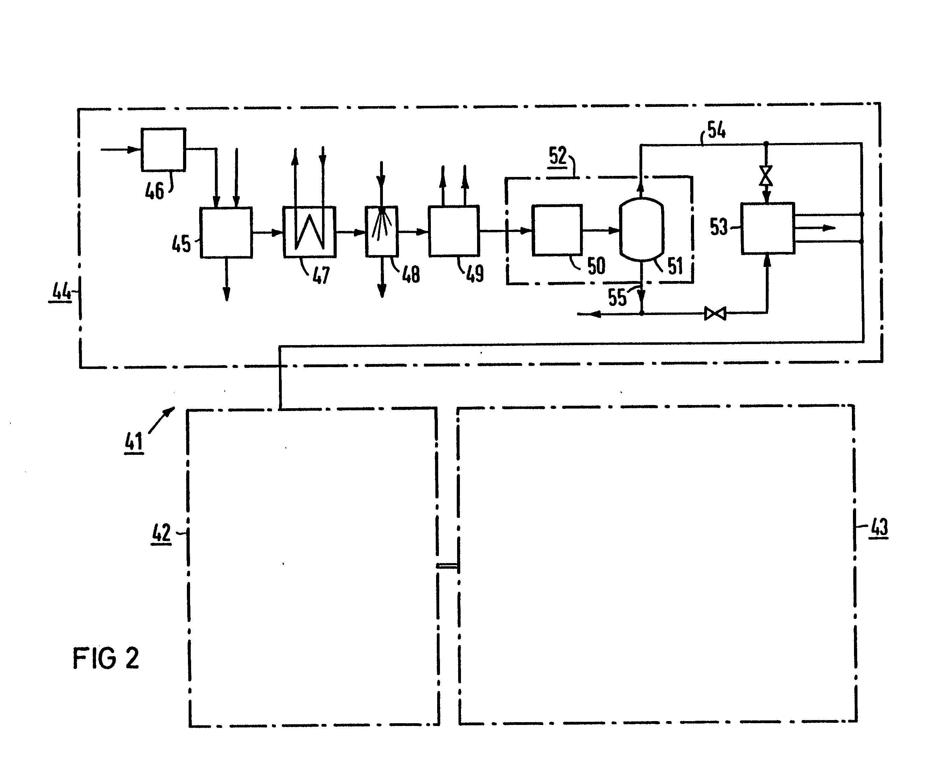

Die Fig. 2 zeigt ein anderes Kraftwerk 41, dessen Gasturbinenkraftwerksteil 42 und Dampfkraftwerksteil 43 identisch mit dem des Ausführungsbeispiels der Fig. 1 ist. Die dem Gasturbinenkraftwerksteil vorgeschaltete Anlage 44 zur Herstellung von Chemierohstoffen weicht jedoch teilweise von jener des Ausführungsbeispiels der Fig. 1 ab. Auch hier ist dem Kohlevergaser 45 eine Luftzerlegungsanlage 46 zugeordnet und werden die Rohgase nacheinander einer Wärmetauschanlage 47, einer Gasentstaubungsanlage 48 und einer Gasreinigungsanlage 49 zugeleitet. Das der Gasreinigungsanlage entströmende Reingas wird einer aus einem Methanol-Synthesereaktor 50 und einem Methanolabscheider 51 bestehenden Durchlauf-Methanolsyntheseanlage 52 zugeleitet. Der Methanolsyntheseanlage 52 ist jedoch eine Anlage 53 zur Erzeugung von Vinylacetat nachgeschaltet. Deren Restgase und flüssigen Rückstände werden dem Gasturbinenkraftwerksteil 42 zur Verbrennung zugeleitet.

Auch hier wird im Kohlevergaser 45 aus gemahlener Kohle mit Hilfe von Sauerstoff und Prozeßdampf Rohgas erzeugt. Dessen Wärme wird in der Wärmeaustauscheranlage 47 zur Erzeugung von Dampf, der wahlweise als Prozeßdampf oder als Speisedampf für den Dampfkraftwerksteil 43 verwendbar ist, genutzt. In der Gasentstaubungsanlage 48 wird das Rohgas von Staubteilchen befreit und zugleich mit Wasserdampf angereichert. In der Gasreinigungsanlage 49 werden Schwefelwasserstoffgas und Kohlendioxyd entfernt. Das verbleibende, im wesentlichen Wasserstoff und Kohlenmonoxyd enthaltene Reingas wird hier in unveränderter Zusammensetzung der Durchlauf-Methanolsyntheseanlage 52 zugeführt. Weil das Verhältnis $H_2$ zu CO im Bereich von 0,5 bis 1 liegt, also noch weit vom stöchiometrischen Verhältnis von 2 entfernt ist, ist der Umsatz zu Methanol noch wesentlich geringer als bei stöchiometrischer Zusammensetzung des Reingases zu erwarten ist. Im anschließenden Methanolabscheider 51 wird das Methanol vom Abgas der Methanolsynthese abgetrennt. Die Abgasleitung 54 und die Methanolausgangsleitung 55 des Methanolabscheiders 51 sind an der Anlage 53 zur Erzeugung von Vinylacetat angeschlossen.

Dem Anschluß einer Anlage zur Erzeugung von Vinylacetat an eine mit Reingas aus einem Kohlevergaser gespeisten Methanolsyntheseanlage 52 kommt es zugute, daß das gesamtstöchiometrische Verhältnis von $H_2$ zu CO für die Erzeugung von Vinylacetat über Methanol aus einem $H_2$ und CO haltigen Ausgangsgas 0,5 beträgt und daher ähnlich der Reingaszusammensetzung ist. Hierdurch wird eine Gaszerlegungsanlage zur Anreicherung des Reingases mit Wasserstoff wie bei der Essigsäuresynthese eingespart. Das überschüssige Methanol steht als verkaufsfähiger Chemierohstoff zur Verfügung. Die Restgase und flüssigen Rückstände der Anlage 53 zur Vinylacetatherstellung sowie die nicht verwendeten Anteile des Syntheseabgases aus dem Methanolabscheider 51 können dem Gasturbinenkraftwerksteil 42 zugeleitet und dort verbrannt werden. Ihre chemisch gebundene Energie geht also nicht verloren. Infolge der Möglichkeit der Verbrennung der Syntheseabgase kann der Aufwand für die Gasreinigung gegenüber bekannten Herstellverfahren verringert werden. Denn dort muß auch der für die Vinylacetaterzeugung abgezweigte Rohgasstrom vollständig von Kohlendioxyd und Schwefelwasserstoff gereinigt werden. Das Vinylacetat kann daher ebenfalls preisgünstiger hergestellt und wie das Methanol als Chemierohstoff verkauft werden.

Mit dieser Kraftwerkskonzeption ist es möglich, das Verhältnis von erzeugter elektrischer Energie und Vinylacetat durch Veränderung der in die Vinylacetatsynthese eingespeisten Gasmenge in gewissen Grenzen an die momentane Bedarfslage anzupassen.

## Patentansprüche

1. Kraftwerk mit einer integrierten Kohlevergasungsanlage, mit einer am Kohlevergaser angeschlossenen Wärmetauscher- und Gasreinigungsanlage, mit einem an die Wärmetauscher- und Gasreinigungsanlage angeschlossenen Gasturbinen- und Dampfkraftwerksteil, und mit einer Methanolsyntheseanlage, wobei das überschüssige Synthesegas aus der Methanolsynthese der Brennkammer des Gasturbinenkraftwerksteils zuleitbar ist, dadurch gekennzeichnet, daß das in der Methanolsyntheseanlage (10, 52) erzeugte Methanol wie auch das Syntheseabgas der Methanolsynthese zumindest teilweise einer Essigsäuresyntheseanlage (13), und das Abgas aus dieser Essigsäuresyntheseanlage (13) der Brennkammer (22) des Gasturbinenkraftwerkteils (2) zuleitbar ist, und eine Gaszerlegungsanlage (17) an der Gaszuleitung (18) zum Methanolsynthesereaktor (11) angeschlossen ist, deren wasserstofffreiche Fraktion dem in den Methanolsynthesereaktor strömenden Gasstrom beimischbar und deren kohlenmonoxydreiche Fraktion sowohl dem Essigsäurereaktor (14) als auch der Brennkammer (22) des Gasturbinenkraftwerkteils (2) zuleitbar ist.

2. Kraftwerk mit einer integrierten Kohlevergasungsanlage, mit einer am Kohlevergaser angeschlossenen Wärmetauscher- und Gasreinigungsanlage, mit einem an die Wärmetauscher- und Gasreinigungsanlage angeschlossenen Gasturbinen- und Dampfkraftwerksteil und mit einer Methanolsyntheseanlage, wobei das überschüssige Syntheseabgas aus der Methanolsynthese der Brennkammer des Gasturbinenkraftwerksteils zuleitbar ist, dadurch gekennzeichnet, daß das in der Methanolsyntheseanlage (10, 52) erzeugte Methanol wie auch das Syntheseabgas der Methanolsynthese zumindest teilweise einer Anlage (53) zur Erzeugung von Vinylacetat und das Abgas aus dieser Anlage (53) zur Erzeugung von Vinylacetat der Brennkammer (22) des Gasturbinenkraftwerkteils (2, 42) zuleitbar ist.

3. Kraftwerk nach Anspruch 1 oder 2, dadurch gekennzeichnet, daß die Methanolsyntheseanlage (10, 52) rezirkulationsfrei, das heißt ohne Rezirkulationsleitung und Kreislaufverdichter, ausgebildet ist.

4. Kraftwerk nach Anspruch 1, dadurch gekennzeichnet, daß dem Methanolsynthesereaktor (11) eine Wasserelektrolyseanlage (39) zugeordnet ist, deren Sauerstoffleitung an den Kohlevergaser (5) und deren Wasserstoffleitung an die zum Methanolsynthesereaktor (11) führende Gaszuleitung (18) angeschlossen ist.

5. Kraftwerk nach Anspruch 1 oder 2, dadurch gekennzeichnet, daß ein Methanolabscheider (12, 51) dem Methanolsynthesereaktor (11, 50) nachgeschaltet ist.

6. Kraftwerk nach Anspruch 1, dadurch gekennzeichnet, daß dem Essigsäurereaktor (14) der Essigsäuresyntheseanlage (13), eine Destillationskolonne (15) für die flüssigen Reaktionsprodukte und ein Waschsystem (16) für die gasförmigen Reaktionsprodukte nachgeschaltet sind.

7. Kraftwerk nach Anspruch 1 oder 2, dadurch gekennzeichnet, daß die dem Rohgas des Kohlevergasers (5, 45) in der Wärmetauscher- und Gasreinigungsanlage (7, 8, 9, 47, 48, 49) entzogene Wärme zumindest teilweise der Erzeugung von Dampf dient.

8. Kraftwerk nach Anspruch 1 oder 2, dadurch gekennzeichnet, daß die in der Methanolsyntheseanlage (10, 52) und in der Essigsäuresyntheseanlage (13) bzw. in der Anlage (53) zur Erzeugung von Vinylacetat frei werdende Wärme zumindest teilweise zur Dampferzeugung herangezogen wird.

9. Kraftwerk nach einem der Ansprüche 1, 2 oder 7, dadurch gekennzeichnet, daß der in der Wärmetauscher- und Gasreinigungsanlage (7, 8, 9, 47, 48, 49) und in der Essigsäuresyntheseanlage (13) bzw. in der Anlage (53) zur Erzeugung von Vinylacetat erzeugte Dampf zusammen mit dem im Abhitzekessel (27) der Gasturbine erzeugten Dampf je nach Betriebszustand des Kraftwerks (1, 41) in unterschiedlichen Anteilen als Betriebsdampf für die Dampfturbine des Dampfkraftwerkteils (3, 43) und als Prozeßdampf für die chemischen Herstellverfahren heranziehbar ist.

## Claims

1. Power station with an integrated coal gasification plant, with a heat exchanger plant and gas purification plant connected to the coal gasifier, with gas turbine- and steam power station sections connected to the heat exchanger- and gas purification plants, and with a methanol synthesis plant, it being possible to supply the superfluous synthesis gas from the methanol synthesis to the combustion chamber of the gas turbine power station section, characterised in that the methanol produced in the methanol synthesis plant (10, 52) and also the synthesis waste gas of the methanol synthesis can be supplied at least partly to an acetic acid synthesis plant (13) and the waste gas from this acetic acid synthesis plant (13) can be supplied to the combustion chamber (22) of the gas turbine power station section (2). and a gas separation plant (17) is connected to the gas supply line (18) to the methanol synthesis reactor (11), the hydrogen rich fraction of which can be mixed with the gas flow flowing into the methanol synthesis reactor and the carbon monoxide rich fraction of which can be supplied to both the acetic acid reactor (14) and to the combustion chamber (22) of the gas turbine power station section (2).

2. Power station with an integrated coal gasification plant, with a heat exchanger plant and gas purification plant connected to the coal gasifier, with gas turbine- and steam power station sections connected to the heat exchanger- and gas purification plants, and with a methanol synthesis plant, it being possible to supply the superfluous synthesis waste gas from the methanol synthesis to the combustion chamber of the gas turbine power station section, characterised in that the methanol produced in the methanol synthesis plant (10, 52) as well as the synthesis waste gas from the methanol synthesis can be supplied at least partly to a plant (53) for producing vinyl acetate and the waste gas from this plant (53) for producing vinyl acetate can be supplied to the combustion chamber (22) of the gas turbine power station section (2, 42).

3. Power station according to claim 1 or 2, charaoterised in that the methanol synthesis plant (10, 52) is designed without recirculation, i. e. without a recirculation line and circuit compressor.

4. Power station according to claim 1, characterised in that a water electrolysis plant (39) is associated with the methanol synthesis reactor (11), having an oxygen line connected to the coal gasifier (5) and a hydrogen line connected to the gas supply line (18) leading to the methanol synthesis reactor (11).

5. Power station according to claim 1 or 2, characterised in that a methanol separator (12, 51) is connected downstream of the methanol synthesis reactor (11, 50).

6. Power station according to claim 1, characterised in that a distillation column (15) for the fluid reaction products and a washing system (16) for the gaseous reaction products are connected downstream of the acetic acid reactor (14) of the acetic acid synthesis plant (13).

7. Power station according to claim 1 or 2, characterised in that the heat extracted from the untreated gas of the coal gasifier (5, 45) in the heat exchanger- and gas purification plants (7, 8, 9, 47, 48, 49) serves at least partly to produce steam.

8. Power station according to claim 1 or 2, characterised in that at least part of the heat being released in the methanol synthesis plant (10, 52) and in the acetic acid synthesis plant (13) or in the plant (53) for producing vinyl acetate is used to produce steam.

9. Power station according to one of the claims 1, 2 or 7, characterised in that the steam produced in the heat exchanger- and gas purification plants (7, 8, 9, 47, 48, 49) and in the acetic acid synthesis plant (13) or in the plant (53) for producing vinyl acetate may be used, together with the steam produced in the waste heat boiler (27) of the gas turbine, according in each case to the operational state of the power station (1, 41) in different proportions as operating steam for the steam turbine of the steam power station section (3, 43) and as process steam for the chemical manufacturing methods.

## Revendications

1. Centrale de production d'électricité, comprenant une installation intégrée de gazéification du charbon, une installation à échangeur de chaleur et d'épuration du gaz reliée au gazogène à charbon, une partie de centrale de production d'électricité à turbine à gaz et à vapeur et une installation de synthèse du méthanol, le gaz de synthèse en excès pouvant être envoyé de la synthèse du méthanol à la chambre de combustion de la partie de centrale de production d'électricité à turbine à gaz, caractérisée en ce que le méthanol produit dans l'installation de synthèse du méthanol (10, 52), tout comme l'effluent gazeux de la synthèse du méthanol, peut être envoyé, au moins en partie, à une installation de synthèse de l'acide acétique (13) et l'effluent gazeux de cette installation de synthèse de l'acide acétique (13) peut être envoyé à la chambre de combustion (22) de la partie de centrale de production d'électricité à turbine à gaz (2) et une installation de fractionnement du gaz (17) est raccordée au conduit (18) d'amenée des gaz au réacteur de synthèse du méthanol (11), sa fraction riche en hydrogène pouvant être mélangée au courant gazeux passant dans le réacteur de synthèse du méthanol et sa fraction riche en monoxyde de carbone pouvant être envoyée tant au réacteur d'acide acétique (14) qu'à la chambre de combustion (22) de la partie de centrale de production d'électricité à turbine à gaz (2).

2. Centrale de production d'électricité, comprenant une installation intégrée de gazéification du charbon, une installation à échangeur de chaleur et d'épuration du gaz reliée au gazogène à charbon, une partie de centrale de production d'électricité à turbine à gaz et à vapeur et une installation de synthèse du méthanol, l'effluent gazeux de synthèse pouvant être envoyé de la synthèse du méthanol à la chambre de combustion de la partie de centrale de production d'électricité à turbine à gaz, caractérisée en ce que le méthanol produit dans l'installation de synthèse du méthanol (10, 52), tout comme l'effluent gazeux de la synthèse du méthanol, peuvent être envoyés, au moins partiellement, à une installation (53) de production d'acétate de vinyle et l'effluent gazeux de cette installation (53) peut être envoyé, pour la production d'acétate de vinyle, à la chambre de combustion (22) de la partie de centrale de production d'électricité à turbine à gaz (2, 42).

3. Centrale de production d'électricité suivant la revendication 1 ou 2, caractérisée en ce que l'installation de synthèse du méthanol (10, 52) n'est pas à recirculation, c'est-à-dire qu'elle n'a ni conduit de recirculation, ni compresseur en circuit fermé.

4. Centrale de production d'électricité suivant la revendication 1, caractérisée en ce qu'au réacteur de synthèse du méthanol (11) est associée une installation d'électrolyse de l'eau (39), dont le conduit pour l'oxygène est raccordé au gazogène à charbon (5) et dont le conduit pour l'hydrogène est raccordé au conduit (18) amenant le gaz au réacteur de synthèse du méthanol (11).

5. Centrale de production d'électricité suivant la revendication 1 ou 2, caractérisée en ce qu'un séparateur de méthanol (12, 51) est monté en aval du réacteur de synthèse de méthanol (11, 50).

6. Centrale de production d'électricité suivant la revendication 1, caractérisée en ce qu'en aval du réacteur d'acide acétique (14) de l'installation de synthèse d'acide acétique (13), sont montées une colonne de distillation (15) pour les produits liquides de la réaction et un système de lavage (16) pour les produits gazeux de la réaction.

7. Centrale de production d'électricité suivant la revendication 1 ou 2, caractérisée en ce que la chaleur prélevée du gaz brut du gazogène à charbon (5, 45) dans l'installation à échangeur de chaleur et d'épuration des gaz (7, 8, 9, 47, 48, 49) sert, au moins en partie, à produire de la vapeur d'eau.

8. Centrale de production d'électricité suivant la revendication 2, caractérisée en ce que la chaleur dégagée dans l'installation de synthèse du méthanol (10, 52) et dans l'installation de synthèse de l'acide acétique (13) ou dans l'installation (53) de production d'acétate de vinyle, est utilisée, au moins en partie, pour produire de la vapeur d'eau.

9. Centrale de production d'électricité suivant l'une des revendications 1, 2 ou 7, caractérisée en ce que la vapeur d'eau produite dans l'installation à échangeur de chaleur et d'épuration des gaz (7, 8, 9, 47, 48, 49) et dans l'installation de synthèse de l'acide acétique (13) ou dans l'installation (53) de production d'acétate de vinyle, est utilisée, en même temps que la vapeur d'eau produite dans la chaudière de récupération (27) de la turbine à gaz, suivant l'état de fonctionnement de la centrale de production d'électricité (1, 41), en des proportions différentes comme vapeur de fonctionnement pour la turbine à vapeur de la partie de centrale de production d'électricité à vapeur (3, 43) et comme vapeur du procédé pour les procédés chimiques de préparation.

FIG 1

EP 0 128 404 B1

FIG 2